# EUROPEAN PATENT SPECIFICATION

(11) **EP 1 383 906 B1**
(45) Date of publication and mention of the grant of the patent: **30.07.2008**
(21) Application number: 02719983.5
(22) Date of filing: 06.03.2002
(51) Int. Cl.: C12P 13/08, C12P 13/04

(54) **PROCESS FOR THE PRODUCTION OF L-AMINO ACIDS USING STRAINS OF THE FAMILY ENTEROBACTERIACEAE THAT CONTAIN AN ATTENUATED DGSA GENE**
VERFAHREN ZUR HERSTELLUNG VON L-AMINOSÄUREN UNTER VERWENDUNG VON STÄMMEN AUS DER FAMILIE DER ENTEROBACTERIACEA DIE EIN ATTENUIERTES DGSA-GEN ENTHALTEN
PROCEDE DE PRODUCTION DE L-AMINO ACIDES AU MOYEN DE SOUCHES DE LA FAMILLE ENTEROBACTERIACEAE CONTENANT UN GENE DGSA ATTENUE

(30) Priority: 03.04.2001 DE 10116518
(43) Date of publication of application: 28.01.2004
(73) Proprietor: Evonik Degussa GmbH, 40474 Düsseldorf (DE)
(72) Inventor: RIEPING, Mechthild, 33619 Bielefeld (DE); HERMANN, Thomas, 33739 Bielefeld (DE)
(86) International application number: PCT/EP2002/002419
(87) International publication number: WO 2002/081721

(56) References cited:
- EP-A- 0 237 819
- EP-A- 0 643 135
- WO-A-99/53035
- WO-A-02/081698
- WO-A-02/081722
- US-A- 4 278 765
- MORRIS P W ET AL.: "Cloning and location of the dgsA gene of Escherichia coli." JOURNAL OF BACTERIOLOGY, vol. 163, no. 2, August 1985 (1985-08), pages 785-786, XP008016939 ISSN: 0021-9193 cited in the application
- HOSONO K ET AL.: "Decreasing accumulation of acetate in a rich medium by Escherichia coli on introduction of genes on a multicopy plasmid." BIOSCIENCE, BIOTECHNOLOGY, AND BIOCHEMISTRY, vol. 59, no. 2, February 1995 (1995-02), pages 256-261, XP001148978 ISSN: 0916-8451 cited in the application
- DATABASE WPI Section Ch, Week 199148 Derwent Publications Ltd., London, GB; Class B05, AN 1991-351136 XP002241222 & JP 03 236786 A (KYOWA HAKKO KOGYO KK), 22 October 1991 (1991-10-22)
- MICHAL G: "Biochemical pathways: an atlas of biochemistry and molecular biology" 1999 , JOHN WILEY & SONS INC. AND SPEKTRUM AKADEMISCHER VERLAG , NEW YORK - HEIDELBERG XP002240848 ISBN: 0-471-33130-9 figures 4.2-1, 4.5-1 and 4.5-2 paragraph [4.5.3]
- JETTEN M S M ET AL.: "Recent advances in the physiology and genetics of amino acid-producing bacteria." CRC CRITICAL REVIEWS IN BIOTECHNOLOGY, vol. 15, no. 1, 1995, pages 73-103, XP000613291 ISSN: 0738-8551
- KRAEMER R: "Genetic and physiological approaches for the production of amino acids" JOURNAL OF BIOTECHNOLOGY, vol. 45, no. 1, 1996, pages 1-21, XP002178648 ISSN: 0168-1656
- SAWERS G: "The anaerobic degradation of L-serine and L-threonine in enterobacteria: networks of pathways and regulatory signals" ARCHIVES OF MICROBIOLOGY, vol. 171, no. 1, 1998, pages 1-5, XP002953871 ISSN: 0302-8933

## Description

### Field of the Invention

The present invention relates to a process for the enzymatic production of the L-amino acids L-threonine, L-lysine and L-valine using strains of the family Enterobacteriaceae in which the dgsA gene is attenuated.

### Prior Art

L-amino acids, in particular L-threonine, are used in human medicine and in the pharmaceutical industry, in the foodstuffs industry, and most especially in animal nutrition.

It is known to produce L-amino acids by fermentation of strains of Enterobacteriaceae, in particular Escherichia coli (E. coli) and Serratia marcescens. On account of their great importance efforts are constantly being made to improve processes for producing the latter. Process improvements may relate to fermentation technology measures, such as for example stirring and provision of oxygen, or the composition of the nutrient media, such as for example the sugar concentration during the fermentation, or the working-up to the product form, for example by ion exchange chromatography, or the intrinsic performance properties of the microorganism itself.

Methods comprising mutagenesis, selection and mutant choice are employed in order to improve the performance properties of these microorganisms. In this way strains are obtained that are resistant to antimetabolites, such as for example the threonine analogue α-amino-β-hydroxyvaleric acid (AHV) or are auxotrophic for regulatorily important metabolites, and that produce L-amino acids such as for example L-threonine.

Methods of recombinant DNA technology have also been used for some years in order to improve strains of the family Enterobacteriaceae producing L-amino acids, by amplifying individual amino acid biosynthesis genes and investigating their effect on production.

### Object of the Invention

The object of the invention is to provide new measures for the improved enzymatic production of L-amino acids, in particular L-threonine.

### Summary of the Invention

The present invention provides a process for the enzymatic production of L-amino acids, chosen from the group L-threonine, L-lysine and L-valine using microorganisms of the family Enterobacteriaceae that in particular already produce the desired L-amino acid and in which the nucleotide sequence coding for the dgsA gene is attenuated.

### Detailed Description of the Invention

Where L-amino acids or amino acids are mentioned hereinafter, this is understood to mean one or more amino acids including their salts, selected from the group comprising L-threonine, L-valine and L-lysine. L-threonine is particularly preferred.

The term "attenuation" describes in this connection the reduction or switching off of the intracellular activity of one or more enzymes (proteins) in a microorganism that are coded by the corresponding DNA, by using for example a weak promoter or a gene or allele that codes for a corresponding enzyme with a low activity and/or that inactivates the corresponding enzyme (protein) or gene, and optionally combining these measures.

By means of these attenuation measures the activity or concentration of the corresponding protein is generally reduced to 0 to 75%, 0 to 50%, 0 to 25%, 0 to 10% or 0 to 5% of the activity or concentration of the wild type protein, or the activity or concentration of the protein in the initial microorganism.

The process is characterized in that the following steps are carried out:
a) fermentation of microorganism of the family Enterobacteriaceae producing the desired L-amino acid,
b) enrichment of the corresponding L-amino acid in the medium or in the cells of the microorganisms of the family Enterobacteriaceae, and
c) isolation of the desired L-amino acid, in which optionally constituents of the fermentation broth and/or the biomass in its entirety or parts thereof remain in the product,
wherein in the microorganism compared to the initial microorganism the expression of the dgsA gene or the expression of nucleotide sequences coding for the gene product of said gene, the regulator of the phosphotransferase system, is switched off.

The microorganisms that are the subject of the present invention can produce L-amino acids from glucose, sucrose, lactose, fructose, maltose, molasses, optionally starch, optionally cellulose or from glycerol and ethanol. The microorganisms are members of the family Enterobacteriaceae selected from the genera Escherichia, Erwinia, Providencia and Serratia. The genera Escherichia and Serratia are preferred. In the case of the genus Escherichia the species Escherichia coli may in particular be mentioned, and in the case of the genus Serratia the species Serratia marcescens may in particular be mentioned.

Suitable strains of the genus Escherichia, in particular those of the species Escherichia coli, that produce in particular L-threonine include for example:
Escherichia coli TF427
Escherichia coli H4578
Escherichia coli KY10935
Escherichia coli VNIIgenetika MG442
Escherichia coli VNIIgenetika M1
Escherichia coli VNIIgenetika 472T23
Escherichia coli BKIIM B-3996
Escherichia coli kat 13
Escherichia coli KCCM-10132

Suitable strains of the genus Serratia, in particular of the species Serratia marcescens, that produce L-threonine include for example:
Serratia marcescens HNr21
Serratia marcescens TLr156
Serratia marcescens T2000

Strains of the family of Enterobacteriaceae producing L-threonine preferably have, *inter alia,* one or more of the genetic or phenotype features selected from the following group: resistance to α-amino-β-hydroxyvaleric acid, resistance to thialysine, resistance to ethionine, resistance to α-methylserine, resistance to diaminosuccinic acid, resistance to α-aminobutyric acid, resistance to borrelidin, resistance to rifampicin, resistance to valine analogues such as for example valine hydroxamate, resistance to purine analogues such as for example 6-dimethylaminopurine, need for L-methionine, optionally partial and compensatable need for L-isoleucine, need for meso-diaminopimelic acid, auxotrophy with regard to threonine-containing dipeptides, resistance to L-threonine, resistance to L-homoserine, resistance to L-lysine, resistance to L-methionine, resistance to L-glutamic acid, resistance to L-aspartate, resistance to L-leucine, resistance to L-phenylalanine, resistance to L-serine, resistance to L-cysteine, resistance to L-valine, sensitivity to fluoropyruvate, defective threonine dehydrogenase, optionally ability to utilise sucrose, enhancement of the threonine operon, enhancement of homoserine dehydrogenase, I-aspartate kinase I, preferably of the feedback-resistant form, enhancement of homoserine kinase, enhancement of threonine synthase, enhancement of aspartate kinase, optionally of the feedback-resistant form, enhancement of aspartate semialdehyde dehydrogenase, enhancement of phosphoenol pyruvate carboxylase, optionally of the feedback-resistant form, enhancement of phosphoenol pyruvate synthase, enhancement of transhydrogenase, enhancement of the RhtB gene product, enhancement of the RhtC gene product, enhancement of the YfiK gene product, enhancement of a pyruvate carboxylase, and attenuation of acetic acid formation.

It has now been found that microorganisms of the family Enterobacteriaceae after attenuation, in particular after switching off the dgsA gene, produce L-amino acids, in particular L-threonine, in an improved way.

The nucleotide sequences of the Escherichia coli genes belong to the prior art and may also be obtained from the genome sequence of Escherichia coli published by Blattner et al. (Science 277, 1453 - 1462 (1997)).

The dgsA gene is described *inter alia* by the following data:
- Designation:: Regulator of the phosphotransferase system
   - EC-No.:: -
   - Reference:: Hosono et al.; Bioscience, Biotechnology and Biochemistry 59, 256-261 (1995) Morris et al.; Journal of Bacteriology 163, 785-786 (1985)
   - Accession No.:: AE000255
   - Note:: The dgsA gene is also designated as mlc gene in the prior art.

Apart from the described dgsA gene, alleles of the gene may be used that result from the degeneracy of the genetic code or from functionally neutral sense mutations, the activity of the protein not being substantially altered.

In order to achieve an attenuation the expression of the gene or the catalytic properties of the enzyme proteins may for example be reduced or switched off. Optionally both measures may be combined.

The gene expression may be reduced by suitable culture conditions, by genetic alteration (mutation) of the signal structures of the gene expression, or also by antisense-RNA techniques. Signal structures of the gene expression are for example repressor genes, activator genes, operators, promoters, attenuators, ribosome-binding sites, the start codon and terminators. The person skilled in the art may find relevant information in, *inter alia,* articles by Jensen and Hammer (Biotechnology and Bioengineering 58: 191-195 (1998)), by Carrier and Keasling (Biotechnology Progress 15, 58-64 (1999), Franch and Gerdes (Current Opinion in Microbiology 3, 159-164 (2000)) and in known textbooks of genetics and molecular biology, such as for example the textbook by Knippers ("Molekulare Genetik", 6th Edition, Georg Thieme Verlag, Stuttgart, Germany, 1995) or that by Winnacker ("Gene and Klone", VCH Verlagsgesellschaft, Weinheim, Germany, 1990).

Mutations that lead to a change or reduction of the catalytic properties of enzyme proteins are known from the prior art. As examples there may be mentioned the work by Qiu and Goodman (Journal of Biological Chemistry 272: 8611-8617 (1997)), Yano et al. (Proceedings of the National Academy of Sciences, USA 95, 5511-5515 (1998), Wente and Schachmann (Journal of Biological Chemistry 266, 20833-20839 (1991). Detailed information may be obtained from known textbooks on genetics and molecular biology, such as for example that by Hagemann ("Allgemeine Genetik", Gustav Fischer Verlag, Stuttgart, 1986).

Suitable mutations include transitions, transversions, insertions and deletions. Depending on the action of the amino acid exchange on the enzyme activity, one speaks of missense mutations or nonsense mutations. Insertions or deletions of at least one base pair in a gene lead to frame shift mutations, which in turn lead to the incorporation of false amino acids or the premature termination of a translation. If as a result of the mutation a stop codon is formed in the coding region, this also leads to a premature termination of the translation. Deletions of several codons typically lead to a complete disruption of the enzyme activity. Details regarding the production of such mutations belong to the prior art and may be obtained from known textbooks on genetics and molecular biology, such as for example the textbook by Knippers ("Molekulare Genetik", 6th Edition, Georg Thieme Verlag, Stuttgart, Germany, 1995), that by Winnacker ("Gene und Klone", VCH Verlagsgesellschaft, Weinheim, Germany, 1990) or that by Hagemann ("Allgemeine Genetik", Gustav Fischer Verlag, Stuttgart, 1986).

Suitable mutations in the genes such as for example deletion mutations may be incorporated by gene and/or allele exchange in suitable strains.

A conventional method is the method of gene exchange by means of a conditionally replicating pSC101 derivate pMAK705 described by Hamilton et al. (Journal of Bacteriology 171, 4617 - 4622 (1989)). Other methods described in the prior art, such as for example that of Martinez-Morales et al. (Journal of Bacteriology 181, 7143-7148 (1999)) or that of Boyd et al.. (Journal of Bacteriology 182, 842-847 (2000)) may likewise be used.

It is also possible to transfer mutations in the respective genes or mutations relating to the expression of the relevant genes, by conjugation or transduction into various strains.

Furthermore for the production of L-threonine, using strains of the family Enterobacteriaceae it may be advantageous in addition to the attenuation of the dgsA gene also to enhance one or more enzymes of the known threonine biosynthesis pathway or enzymes of anaplerotic metabolism or enzymes for the production of reduced nicotinamide-adenine-dinucleotide phosphate.

The term "enhancement" describes in this connection the raising of the intracellular activity of one or more enzymes or proteins in a microorganism that are coded by the corresponding DNA, by for example increasing the number of copies of the gene or genes, using a strong promoter or a gene that codes for a corresponding enzyme or protein having a high activity, and optionally by combining these measures.

By means of the aforementioned enhancement measures, in particular overexpression, the activity or concentration of the corresponding protein is in general raised by at least 10%, 25%, 50%, 75%, 100%, 150%, 200%, 300%, 400% or 500%, at most up to 1000% or 2000% referred to that of the wild type protein and/or the activity or concentration of the protein in the initial microorganism.

Thus, one or more of the genes selected from the following group may for example be simultaneously overexpressed:
- the thrABC operon coding for aspartate kinase, homoserine dehydrogenase, homoserine kinase and threonine synthase (US-A-=4, 278,765),
- the pyc gene coding for pyruvate carboxylase (DE-A-19 831 609),
- the pps gene coding for phosphoenol pyruvate synthase (Molecular and General Genetics 231:332 (1992)),
- the ppc gene coding for phosphoenol pyruvate carboxylase (Gene 31:279-283 (1984)),
- the genes pntA and pntB coding for transhydrogenase (European Journal of Biochemistry 158:647-653 (1986)),
- the gene rhtB imparting homoserine resistance (EP-A-0 994 190),
- the mqo gene coding for malate:quinone oxidoreductase (DE 100 348 33.5),
- the gene rhtC imparting threonine resistance (EP-A-1 013 765), and
- the thrE gene of Corynebacterium glutamicum coding for threonine export (DE 100 264 94.8).

The use of endogenous genes is in general preferred. The term "endogenous genes" or "endogenous nucleotide sequences" is understood to mean the genes or nucleotide sequences present in the population of a species.

Furthermore for the production of L-threonine, it may be advantageous in addition to the attenuation of the dgsA gene also to attenuate, in particular to switch off or reduce the expression of one or more of the genes selected from the following group:
- the tdh gene coding for threonine dehydrogenase (Ravnikar and Somerville, Journal of Bacteriology 169, 4716-4721 (1987)),
- the mdh gene coding for malate dehydrogenase (E.C. 1.1.1.37) (Vogel et al., Archives in Microbiology 149, 36-42 (1987)),
- the gene product of the open reading frame (orf) yjfA (Accession Number AAC77180 of the National Center for Biotechnology Information (NCBI, Bethesda, MD, USA),
- the gene product of the open reading frame (orf) ytfP (Accession Number AAC77179 des National Center for Biotechnology Information (NCBI, Bethesda, MD, USA),
- the pckA gene coding for the enzyme phosphoenol pyruvate carboxykinase (Medina et al. (Journal of Bacteriology 172, 7151-7156 (1990)),
- the poxB gene coding for pyruvate oxidase (Grabau and Cronan (Nucleic Acids Research 14 (13), 5449-5460 (1986)),
- the fruR gene coding for the fructose repressor: (Jahreis et al., Molecular and General Genetics 226, 332-336 (1991) and Accession No.: AE000118),and
- the aceA gene for isocitrate lyase (EC-No.: 4.1.3.1) kodierende (Matsuoko and McFadden; Journal of Bacteriology 170, 4528-4536 (1988) and Accession No.: AE000474)

Furthermore for the production of L-amino acids, in particular L-threonine, it may be advantageous in addition to the attenuation of the dgsA gene also to switch off undesirable secondary reactions (Nakayama: "Breeding of Amino Acid Producing Microorganisms", in: Overproduction of Microbial Products, Krumphanzl, Sikyta, Vanek (eds.), Academic Press, London, UK, 1982).

The microorganisms produced according to the invention may be cultivated in a batch process (batch cultivation), in a fed batch process (feed process) or in a repeated fed batch process (repetitive feed process). A summary of known cultivation methods is described in the textbook by Chmiel (Bioprozesstechnik 1. Einführung in die Bioverfahrenstechnik (Gustav Fischer Verlag, Stuttgart, 1991)) or in the textbook by Storhas (Bioreaktoren and periphere Einrichtungen (Vieweg Verlag, Brunswick/Wiesbaden, 1994)).

The culture medium to be used must appropriately satisfy the requirements of the respective strains. Descriptions of culture media of various microorganisms are contained in the handbook "Manual of Methods for General Bacteriology" of the American Society for Bacteriology (Washington D.C., USA, 1981).

As carbon sources, sugars and carbohydrates such as for example glucose, sucrose, lactose, fructose, maltose, molasses, starch and optionally cellulose, oils and fats such as for example soya bean oil, sunflower oil, groundnut oil and coconut oil, fatty acids such as for example palmitic acid, stearic acid and linoleic acid, alcohols such as for example glycerol and ethanol, and organic acids such as for example acetic acid, may be used. These substances may be used individually or as a mixture.

As nitrogen source, organic nitrogen-containing compounds such as peptones, yeast extract, meat extract, malt extract, maize starch water, soya bean flour and urea or inorganic compounds such as ammonium sulfate, ammonium chloride, ammonium phosphate, ammonium carbonate and ammonium nitrate may be used. The nitrogen sources may be used individually or as a mixture.

As phosphorus source, phosphoric acid, potassium dihydrogen phosphate or dipotassium hydrogen phosphate or the corresponding sodium-containing salts may be used. The culture medium must furthermore contain salts of metals, such as for example magnesium sulfate or iron sulfate, that are necessary for growth. Finally, essential growth promoters such as amino acids and vitamins may be used in addition to the aforementioned substances. Apart from these, suitable precursors may be added to the culture medium. The aforementioned starting substances may be added to the culture in the form of a single batch or may be metered in in an appropriate manner during the cultivation.

In order to regulate the pH of the culture basic compounds such as sodium hydroxide, potassium hydroxide, ammonia or ammonia water, or acidic compounds such as phosphoric acid or sulfuric acid are used as appropriate. In order to control foam formation antifoaming agents such as for example fatty acid polyglycol esters may be used. In order to maintain the stability of plasmids, suitable selectively acting substances, for example antibiotics, may be added to the medium. In order to maintain aerobic conditions, oxygen or oxygen-containing gas mixtures such as for example air are fed into the culture. The temperature of the culture is normally 25°C to 45°C, and preferably 30°C to 40°C. Cultivation is continued until a maximum amount of L-amino acids (or L-threonine) has been formed. This target is normally achieved within 10 hours to 160 hours.

The L-amino acids may be analyzed by anion exchange chromotography followed by ninhydrin derivation, as described by Spackman et al. (Analytical Chemistry, 30, (1958), 1190), or by reversed phase HPLC, as described by Lindroth et al. (Analytical Chemistry (1979) 51: 1167-1174).

The process according to the invention can be used for the enzymatic production of the L-amino acids L-threonine, L-valine and L-lysine, in particular L-threonine.

A pure culture of the Escherichia coli K-12 strain DH5α/pMAK705 was filed as DSM 13720 on 08 September 2000 at the German Collection for Microorganisms and Cell Cultures (DSMZ, Brunswick, Germany) according to the Budapest Convention.

The present invention is described in-more detail hereinafter with the aid of examples of implementation.

The isolation of plasmid DNA from Escherichia coli as well as all techniques for the restriction, Klenow treatment and alkaline phosphatase treatment are carried out according to Sambrook et al. (Molecular Cloning - A Laboratory Manual (1989) Cold Spring Harbor Laboratory Press). The transformation of Escherichia coli is, unless otherwise described, carried out according to Chung et al. (Proceedings of the National Academy of Sciences of the United States of America, USA (1989) 86: 2172-2175).

The incubation temperature in the production of strains and transformants is 37°C. In the gene exchange process according to Hamilton et al., temperatures of 30°C and 44°C are used.

### Example 1

### Construction of the deletion mutation of the dgsA gene

Parts of the gene regions lying upstream and downstream of the dgsA gene and parts of the 5'-region and 3'-region of the dgsA gene are amplified from Escherichia coli K12 using the polymerase chain reaction (PCR) as well as synthetic oligonucleotides. Starting from the nucleotide sequence of the dgsA gene and sequences in E. coli K12 MG1655 (SEQ ID No. 1, Accession Number AE000255) lying upstream and downstream, the following PCR primers are synthesised (MWG Biotech, Ebersberg, Germany):
dgsA'5'-1: 5' - CGAATGTAACGCTGGCTGAA - 3' (SEQ ID No. 3)
dgsA'5'-2: 5' - TCCAGCAATGGCAAGTCATC - 3' (SEQ ID No. 4)
dgsA'3'-1: 5' - CAGCACATCAGCGTTGAGAG - 3' (SEQ ID No. 5)
dgsA'3'-2: 5' - GATCGCCTGAGCTGTTAGCA - 3' (SEQ ID No. 6)

The chromosomal E. coli K12 MG1655 DNA used for the PCR is isolated according to the manufacturer's instructions using "Qiagen Genomic-tips 100/G" (QIAGEN, Hilden, Germany). A ca. 850 bp large DNA fragment from the 5'-region of the dgsA gene region (designated dgsA1) and a ca. 700 bp large DNA fragment from the 3'-region of the dgsA gene region (designated dgsA2) can be amplified with the specific primers under standard PCR conditions (Innis et al. (1990) PCR Protocols. A Guide to Methods and Applications, Academic Press) with taq-DNA-polymerase (Gibco-BRL, Eggenstein, Germany). The PCR products are ligated according to the manufacturer's instructions in each case with the vector pCR2.1TOPO (TOPO TA Cloning Kit, Invitrogen, Groningen, Netherlands) and transformed in the E. coli strain TOP10F'. The selection of plasmid-carrying cells is carried out on LB agar to which 50 µg/ml of ampicillin has been added. After the plasmid DNA isolation the vector pCR2.1TOPOdgsA2 is cleaved with the restriction enzymes Ecl136II and XbaI, and the dgsA2 fragment after separation in 0.8% agarose gel is isolated using the QIAquick Gel Extraction Kit (QIAGEN, Hilden, Germany). After the plasmid DNA isolation the vector pCR2.1TOPOdgsAl is cleaved with the enzymes EcoRV and XbaI and ligated with the isolated dgsA2 fragment. The E. coli strain DH5α is transformed with the ligation batch and plasmid-carrying cells are selected on LB agar to which 50 µg/ml of ampicillin has been added. After the plasmid DNA isolation, those plasmids in which the mutagenic DNA sequence shown in SEQ ID No. 7 is present in cloned form are detected by control cleavage with the enzymes HindIII and XbaI. One of the plasmids is designated pCR2.1TOPOΔdgsA.

### Example 2

### Construction of the exchange vector pMAK705ΔdgsA

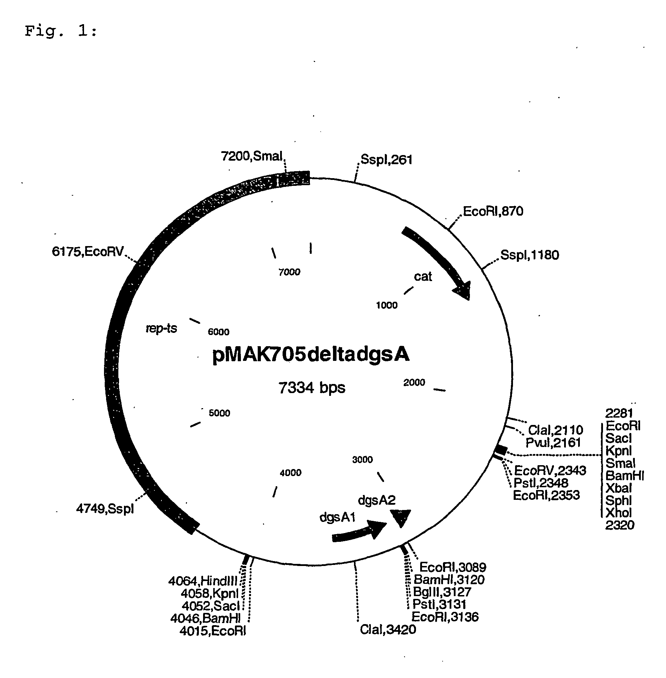

The dgsA allele described in Example 1 is isolated from the vector pCR2.1TOPOΔdgsA after restriction with the enzymes HindIII and XbaI and separation in 0.8% agarose gel, and is ligated with the plasmid pMAK705 (Hamilton et al. (1989) Journal of Bacteriology 171, 4617 - 4622), that had been digested with the enzymes HindIII and XbaI. The ligation batch is transformed in DH5α and plasmid-carrying cells are selected on LB agar to which 20 µg/ml of chloramphenicol have been added. The successful cloning is detected after plasmid DNA isolation and cleavage with the enzymes HindIII and XbaI. The resultant exchange vector pMAK705ΔdgsA (= pMAK705deltadgsA) is shown in Fig. 1.

### Example 3

### Site-specific mutagenesis of the dgsA gene in the E. coli strain MG442

The E. coli strain MG442 producing L-threonine is described in patent specification US-A- 4,278,765 and is filed as CMIM B-1628 at the Russian National Collection for Industrial Microorganisms (VKPM, Moscow, Russia).

For the exchange of the chromosomal dgsA gene by the plasmid-coded deletion construct, MG442 is transformed with the plasmid pMAK705ΔdgsA. The gene exchange is carried out by the selection process described by Hamilton et al. (1989) Journal of Bacteriology 171, 4617 - 4622) and is verified by standard PCR methods (Innis et al. (1990) PCR Protocols. A guide to methods and applications, Academic Press) with the following oligonucleotide primers:
dgsA'5'-1: 5' - CGAATGTAACGCTGGCTGAA - 3' (SEQ ID No. 3)
dgsA'3'-2: 5' - GATCGCCTGAGCTGTTAGCA - 3' (SEQ ID No. 6)

After the exchange the form of the ΔdgsA allele shown in SEQ ID No. 8 is present in MG442. The strain obtained is designated MG442ΔdgsA.

### Example 4

### Production of L-threonine using the strain MG442ΔdgsA

MG442ΔdgsA is cultivated on minimal medium having the following composition: 3.5 g/l Na₂HPO₄·2H₂O, 1.5 g/l KH₂PO₄, 1 g/l NH₄Cl, 0.1 g/l MgSO₄·7H₂O, 2 g/l glucose and 20 g/l agar. The formation of L-threonine is checked in batch cultures of 10 ml that are contained in 100 ml Erlenmeyer flasks. For this, 10 ml of preculture medium of the following composition: 2 g/l yeast extract, 10 g/l (NH₄)₂SO₄, 1 g/l KH₂PO₄, 0.5 g/l MgSO₄·7H₂O, 15 g/l CaCO₃, 20 g/l glucose are inoculated and incubated for 16 hours at 37°C and 180 rpm in an ESR incubator from Kühner AG (Birsfelden, Switzerland). 250 µl of this preculture are reinoculated in 10 ml of production medium (25 g/l (NH₄)₂SO₄, 2 g/l KH₂PO₄, 1 g/l MgSO₄·7H₂O, 0.03 g/l FeSO₄·7H₂O, 0.018 g/l MnSO₄·1H₂O, 30 g/l CaCO₃ and 20 g/l glucose) and incubated for 48 hours at 37°C. After incubation the optical density (OD) of the culture suspension is measured with an LP2W photometer from the Dr. Lange company (Dusseldorf, Germany) at a measurement wavelength of 660 nm.

The concentration of formed L-threonine is then determined in the sterile-filtered culture supernatant using an amino acid analyzer from Eppendorf-BioTronik (Hamburg, Germany) by ion exchange chromatography and post-column reaction with ninhydrin detection.

The result of the test is given in Table 1.

**Table 1**

| Strain | OD (660 nm) | L-threonine |
|---|---|---|
| MG442 | 6.0 | 1.5 |
| MG442ΔdgsA | 6.5 | 1.8 |

Brief Description of the Figure:
- Fig. 1: pMAK705ΔdgsA (= pMAK705deltadgsA)
   Length data are given as approximate values. The abbreviations and acronyms used have the following meanings:
- cat: chloramphenicol resistance gene
- rep-ts: temperature-sensitive replication region of the plasmid pSC101
- dgsA1: part of the 5'-region of the dgsA gene and of the upstream-lying region
- dgsA2: part of the 3'-region of the dgsA gene and of the downstream-lying region

The abbreviations for the restriction enzymes have the following meanings:
- BamHI: restriction endonuclease from Bacillus amyloliquefaciens
- BglII: restriction endonuclease from Bacillus globigii
- ClaI: restriction endonuclease from Caryphanon latum
- EcoRI: restriction endonuclease from Escherichia coli
- EcoRV: restriction endonuclease from Escherichia coli
- HindIII: restriction endonuclease from Haemophilus influenzae
- KpnI: restriction endonuclease from Klebsiella pneumoniae
- PstI: restriction endonuclease from Providencia stuartii
- PvuI: restriction endonuclease from Proteus vulgaris
- SacI: restriction endonuclease from Streptomyces achromogenes
- SalI: restriction endonuclease from Streptomyces albus
- SmaI: restriction endonuclease from Serratia marcescens
- SphI: restriction endonuclease from Streptomyces phaeochromogenes
- SspI: restriction endonuclease from Sphaerotilus species
- XbaI: restriction endonuclease from Xanthomonas badrii
- XhoI: restriction endonuclease from Xanthomonas holcicola

## Claims

1. Process for the production of L-amino-acids, chosen from the group L-threonine, L-lysine and L-valine, wherein the following steps are carried out:
a) fermentation of microorganisms of the family Enterobacteriaceae producing the desired L-amino acid,
b) enrichment of the L-amino acid in the medium or in the cells of the microorganisms, and
c) isolation of the L-amino acid, in which optionally constituents of the fermentation broth and/or the biomass in its entirety or portions thereof remain in the product,
wherein in the microorganism compared to the initial microorganism the expression of the dgsA gene or the expression of nucleotide sequences coding for the gene product of said gene, the regulator of the phosphotransferase system, is switched off.

2. Process according to claim 1, wherein, for the production of L-threonine, microorganisms of the family Enterobacteriaceae are fermented in which at the same time one or more of the genes selected from the following group is or are over-expressed:
2.1 the thrABC operon coding for aspartate kinase, homoserine dehydrogenase, homoserine kinase and threonine synthase,
2.2 the pyc gene coding for pyruvate carboxylase,
2.3 the pps gene coding for phosphoenol pyruvate synthase,
2.4 the ppc gene coding for phosphoenol pyruvate carboxylase,
2.5 the genes pntA and pntB coding for transhydrogenase,
2.6 the Escherichia coli rhtB gene coding for a protein imparting homoserine resistance,
2.7 the mqo gene coding for malate:quinone oxidoreductase,
2.8 the Escherichia coli rhtC gene coding for a protein imparting threonine resistance, and
2.9 the thrE gene coding for threonine export.

3. Process according to claim 1, wherein, for the production of L-threonine, microorganisms of the family Enterobacteriaceae are fermented in which at the same time the expression of one or more of the genes selected from the following group is or are switched off:
3.1 the tdh gene coding for threonine dehydrogenase,
3.2 the mdh gene coding for malate dehydrogenase,
3.3 the gene product of the open reading frame (orf) yjfA of E. coli, when said microorganism is Escherichia coli,
3.4 the gene product of the open reading frame (orf) ytfP of E. coli, when said microorganism is Escherichia coli,
3.5 the pckA gene coding for phosphoenol pyruvate carboxykinase, and
3.6 the poxB gene coding for pyruvate oxidase, and
3.7 the aceA gene coding for isocitrate lyase.

4. Recombinant microorganisms of the Enterobacteriaceae family, which produce L-threonine, and wherein at least:
a) the thrABC operon which genes code for aspartate kinase, homoserine dehydrogenase, homoserine kinase and threonine synthase is over-expressed, and
b) the expression of the dgsA gene coding for the regulator of the phosphotransferase system or nucleotide sequences, which code for the dgsA-gene product
is switched off.

5. Recombinant microorganisms of the Enterobacteriaceae family, which produce L-lysine, and wherein at least:
a) the expression of the pckA gene coding for phosphoenol pyruvate carboxykinase is switched off, and
b) the expression of the dgsA gene coding for the regulator of the phosphotransferase system or nucleotide sequences, which code for the dgsA-gene product is switched off.

6. Recombinant microorganisms of the Enterobacteriaceae family, which produce L-valine, and wherein at least:
a) the expression of the poxB gene coding for pyruvate oxidase is switched off, and
b) the expression of the dgsA gene coding for the regulator of the phosphotransferase system or nucleotide sequences, which code for the dgsA-gene product is switched off.

7. Microorganisms according to claim 4 to 6, wherein the microorganisms originate from the genus Escherichia.

8. Microorganisms according to claim 7, wherein the microorganisms originate from the species E. coli.

## Patentansprüche

1. Verfahren zur Herstellung von L-Aminosäuren, ausgewählt aus der Gruppe L-Threonin, L-Lysin und L-Valin, bei dem man die folgenden Schritte durchführt:
a) Fermentation von Mikroorganismen der Familie Enterobacteriaceae, die die gewünschte L-Aminosäure produzieren,
b) Anreicherung der L-Aminosäure im Medium oder in den Zellen der Mikroorganismen und
c) Isolierung der L-Aminosäure, wobei gegebenenfalls Bestandteile der Fermentationsbrühe und/oder die Biomasse insgesamt oder teilweise im Produkt verbleiben,
wobei die Expression des dgsA-Gens oder die Expression der für das Genprodukt des Gens, den Regulator des Phosphotransferase-Systems, codierenden Nukleotidsequenzen in dem Mikroorganismus im Vergleich zu dem ursprünglichen Mikroorganismus ausgeschaltet ist.

2. Verfahren gemäß Anspruch 1, wobei man zur Herstellung von L-Threonin Mikroorganismen der Familie Enterobacteriaceae fermentiert, in denen gleichzeitig eines oder mehrere der Gene, ausgewählt aus der folgenden Gruppe, überexprimiert wird bzw. werden:
2.1 das für Aspartatkinase, Homoserin-Dehydrogenase, Homoserinkinase und Threonin-Synthase codierende thrABC-Operon,
2.2 das für die Pyruvat-Carboxylase codierende pyc-Gen,
2.3 das für die Phosphoenolpyruvat-Synthase codierende pps-Gen,
2.4 das für die Phosphoenolpyruvat-Carboxylase codierende ppc-Gen,
2.5 die für Transhydrogenase codierenden Gene pntA und pntB,
2.6 das für ein Homoserin-Resistenz verleihendes Protein codierende rhtB-Gen aus Escherichia coli,
2.7 das für die Malat:Chinon-Oxidoreduktase codierende mqo-Gen,
2.8 das für ein Threonin-Resistenz verleihendes Protein codierende rhtC-Gen aus Escherichia coli und
2.9 das für Threonin-Export codierende thrE-Gen.

3. Verfahren gemäß Anspruch 1, wobei man zur Herstellung von L-Threonin Mikroorganismen der Familie Enterobacteriaceae fermentiert, in denen gleichzeitig die Expression eines oder mehrerer Gene, ausgewählt aus der nachfolgenden Gruppe, ausgeschaltet ist:
3.1 das für die Threonin-Dehydrogenase codierende tdh-Gen,
3.2 das für die Malat-Dehydrogenase codierende mdh-Gen,
3.3 das Genprodukt des offenen Leserasters (orf) yjfA aus E. coli, wenn es sich bei dem Mikroorganismus um Escherichia coli handelt,
3.4 das Genprodukt des offenen Leserasters (orf) ytfP aus E. coli, wenn es sich bei dem Mikroorganismus um Escherichia coli handelt,
3.5 das für die Phosphoenolpyruvat-Carboxykinase codierende pckA-Gen und
3.6 das für die Pyruvat-Oxidase codierende poxB-Gen und
3.7 das für die Isocitrat-Lyase codierende aceA-Gen.

4. Rekombinante Mikroorganismen der Familie Enterobacteriaceae, die L-Threonin produzieren, wobei wenigstens:
a) das thrABC-Operon, dessen Gene für Aspartatkinase, Homoserin-Dehydrogenase, Homoserinkinase und Threonin-Synthase codieren, überexprimiert wird und
b) die Expression des für den Regulator des Phosphotransferase-Systems codierenden dgsA-Gens oder von für das dgsA-Genprodukt codierenden Nukleotidsequenzen ausgeschaltet ist.

5. Rekombinante Mikroorganismen der Familie Enterobacteriaceae, die L-Lysin produzieren, wobei wenigstens:
a) die Expression des für die Phosphoenolpyruvat-Carboxykinase codierenden pckA-Gens sowie
b) die Expression des für den Regulator des Phosphotransferase-Systems codierenden dgsA-Gens oder von für das dgsA-Genprodukt codierenden Nukleotidsequenzen ausgeschaltet ist.

6. Rekombinante Mikroorganismen der Familie Enterobacteriaceae, die L-Valin produzieren, wobei wenigstens:
a) die Expression des für die Pyruvat-Oxidase codierenden poxB-Gens sowie
b) die Expression des für den Regulator des Phosphotransferase-Systems codierenden dgsA-Gens oder von für das dgsA-Genprodukt codierenden Nukleotidsequenzen ausgeschaltet ist.

7. Mikroorganismen gemäß Ansprüchen 4 bis 6, wobei die Mikroorganismen aus der Gattung Escherichia stammen.

8. Mikroorganismen gemäß Anspruch 7, wobei die Mikroorganismen aus der Spezies E. coli stammen.

## Revendications

1. Procédé de production d'acides L-aminés, choisis dans le groupe comprenant la L-thréonine, la L-lysine et la L-valine, **caractérisé en ce que** les étapes suivantes sont effectuées :
a) la fermentation de microorganismes de la famille des Enterobacteriaceae produisant l'acide L-aminé désiré,
b) l'enrichissement de l'acide L-aminé dans le milieu ou dans les cellules des microorganismes, et
c) l'isolement de l'acide L-aminé, où éventuellement les constituants du bouillon de fermentation et/ou la biomasse dans son intégralité ou des portions de celle-ci restent dans le produit,
**en ce que**, dans le microorganisme et au microorganisme initial, l'expression du gène comparé dgsA ou l'expression des séquences nucléotidiques codant pour le produit génique dudit gène, le régulateur du système phosphotransférase, est éteinte.

2. Procédé selon la revendication 1, **caractérisé en ce que**, pour la production de la L-thréonine, des microorganismes de la famille des Enterobacteriaceae sont mis en fermentation, dans lesquels, en même temps, un ou plusieurs des gènes choisis dans le groupe suivant sont surexprimés :
2.1 l'opéron thrABC codant pour l'aspartate kinase, l'homosérine déshydrogénase, l'homosérine kinase et la thréonine synthase,
2.2 le gène pyc codant pour la pyruvate carboxylase,
2.3 le gène pps codant pour la phosphoénol pyruvate synthase,
2.4 le gène ppc codant pour la phosphoénol pyruvate carboxylase,
2.5 les gènes pntA et pntB codant pour la transhydrogénase,
2.6 le gène rhtB d'Escherichia coli codant pour une protéine donnant la résistance à l'homosérine,
2.7 le gène mqo codant pour la malate:quinone oxydoréductase,
2.8 le gène rhtC d'Escherichia coli codant pour une protéine donnant la résistance à la thréonine, et
2.9 le gène thrE codant pour l'exportation de la thréonine.

3. Procédé selon la revendication 1, **caractérisé en ce que**, pour la production de la L-thréonine, des microorganismes de la famille des Enterobacteriaceae sont mis en fermentation, dans lesquels, en même temps, l'expression d'un ou plusieurs des gènes choisis dans le groupe suivant est éteinte :
3.1 le gène tdh codant pour la thréonine déshydrogénase,
3.2 le gène mdh codant pour la malate déshydrogénase,
3.3 le produit du gène du cadre de lecture ouvert (orf) yjfA d'E. coli, lorsque ledit microorganisme est l'Escherichia coli,
3.4 le produit du gène du cadre de lecture ouvert (orf) ytfP d'E. coli, lorsque ledit microorganisme est l'Escherichia coli,
3.5 le gène pckA codant pour la phosphoénol pyruvate carboxykinase, et
3.6 le gène poxB codant pour la pyruvate oxydase.
3.7 le gène aceA codant pour l'isocitrate lyase.

4. Microorganismes recombinants de la famille des Enterobacteriaceae, produisant la L-thréonine, **caractérisés en ce qu'**au moins :
a) l'opéron thrABC, dont les gènes codent pour l'aspartate kinase, l'homosérine déshydrogénase, l'homosérine kinase et la thréonine synthase, est surexprimé, et
b) l'expression du gène dgsA codant pour le régulateur du système phosphotransférase ou des séquences nucléotidiques codant pour le produit du gène dgsA est éteinte.

5. Microorganismes recombinants de la famille des Enterobacteriaceae, produisant la L-lysine, **caractérisés en ce qu'**au moins :
a) l'expression du gène pckA codant pour la phosphoénol pyruvate carboxykinase est éteinte, et
b) l'expression du gène dgsA codant pour le régulateur du système phosphotransférase ou des séquences nucléotidiques codant pour le produit du gène dgsA est éteinte.

6. Microorganismes recombinants de la famille des Enterobacteriaceae, produisant la L-valine, **caractérisés en ce qu'**au moins :
a) l'expression du gène poxB codant pour la pyruvate oxidase est éteinte, et
b) l'expression du gène dgsA codant pour le régulateur du système phosphotransférase ou des séquences nucléotidiques codant pour le produit du gène dgsA est éteinte.

7. Microorganismes selon les revendications 4 a 6, **caractérisés en ce que** les microorganismes proviennent du genre Escherichia.

8. Microorganismes selon la revendication 7, **caractérisés en ce que** les microorganismes proviennent de l'espèce E. coli.


### SEQUENCE LISTING

<110> Degussa AG
<120> Process for the production of L-amino acids using strains of the family Enterobacteriaceae containing an attenuated dgsA-Gen
<130> 020003 BT
<160> 8
<170> PatentIn version 3.1
<210> 1
   <211> 2306
   <212> DNA
   <213> Escherichia coli
<220>
   <221> CDS
   <222> (485)..(1705)
   <223> dgsA gene
<400> 1
   cgaatgtaac gctggctgaa ctggcgaaag aaccctttgt cttttttgat ccgcacgtcg      60
   ggacagggct gtatgacgat attctcgggc tgatgcgacg ttaccatttg acgcccgtca    120
   tcactcagga ggtgggcgag gcaatgacca tcatcggtct ggtttccgcc ggtctgggtg    180
   tttcaatttt gcctgcgtca tttaaacgtg ttcagctcaa cgaaatgcgc tgggtgccga    240
   ttgctgaaga ggatgcggtt tctgaaatgt ggttggtctg gccgaaacat catgaacaaa    300
   gtccggctgc gcgtaacttt cgtattcatc tgctgaatgc tctcaggtga gggaaatttc    360
   agcgaaaaag cccgaaaaat gtgctgttaa tcacatgcct aagtaaaaat ttgacgacac    420
   gtattgaagt gcttcaccat agcctacaga ttatttcgga gcgcgaaaat atagggagta    480
   tgcg gtg gtt gct gaa aac cag cct ggg cac att gat caa ata aag cag      529
        Met Val Ala Glu Asn Gln Pro Gly His Ile Asp Gln Ile Lys Gln
        1               5                   10                  15
   acc aac gcg ggc gcg gtt tat cgc ctg att gat cag ctt ggt cca gtc      577
   Thr Asn Ala Gly Ala Val Tyr Arg Leu Ile Asp Gln Leu Gly Pro Val
                   20                  25                  30
   tcg cgt atc gat ctt tcc cgt ctg gcg caa ctg gct cct gcc agt atc      625
   Ser Arg Ile Asp Leu Ser Arg Leu Ala Gln Leu Ala Pro Ala Ser Ile
               35                  40                  45
   act aaa att gtc cgt gag atg ctc gaa gca cac ctg gtg caa gag ctg      673
   Thr Lys Ile Val Arg Glu Met Leu Glu Ala His Leu Val Gln Glu Leu
           50                  55                  60
   gaa atc aaa gaa gcg ggg aac cgt ggc cgt ccg gcg gtg ggg ctg gtg      721
   Glu Ile Lys Glu Ala Gly Asn Arg Gly Arg Pro Ala Val Gly Leu Val
       65                  70                  75
   gtt gaa act gaa gcc tgg cac tat ctt tct ctg cgc att agt cgc ggg      769
   Val Glu Thr Glu Ala Trp His Tyr Leu Ser Leu Arg Ile Ser Arg Gly
   80                  85                  90                  95
   gag att ttc ctt gct ctg cgc gat ctg agc agc aaa ctg gtg gtg gaa      817
   Glu Ile Phe Leu Ala Leu Arg Asp Leu Ser Ser Lys Leu Val Val Glu
                   100                 105                 110
   gag tcg cag gaa ctg gcg tta aaa gat gac ttg cca ttg ctg gat cgt      865
   Glu Ser Gln Glu Leu Ala Leu Lys Asp Asp Leu Pro Leu Leu Asp Arg
               115                 120                 125
   att att tcc cat atc gat cag ttt ttt atc cgc cac cag aaa aaa ctt      913
   Ile Ile Ser His Ile Asp Gln Phe Phe Ile Arg His Gln Lys Lys Leu
           130                 135                 140
   gag cgt cta act tcg att gcc ata acc ttg ccg gga att att gat acg      961
   Glu Arg Leu Thr Ser Ile Ala Ile Thr Leu Pro Gly Ile Ile Asp Thr
       145                 150                 155
   gaa aat ggt att gta cat cgc atg ccg ttc tac gag gat gta aaa gag     1009
   Glu Asn Gly Ile Val His Arg Met Pro Phe Tyr Glu Asp Val Lys Glu
   160                 165                 170                 175
   atg ccg ctc ggc gag gcg ctg gag cag cat acc ggc gtt ccg gtt tat     1057
   Met Pro Leu Gly Glu Ala Leu Glu Gln His Thr Gly Val Pro Val Tyr
                   180                 185                 190
   att cag cat gat atc agc gca tgg acg atg gca gag gcc ttg ttt ggt     1105
   Ile Gln His Asp Ile Ser Ala Trp Thr Met Ala Glu Ala Leu Phe Gly
               195                 200                 205
   gcc tca cgc ggg gcg cgc gat gtg att cag gtg gtt atc gat cac aac     1153
   Ala Ser Arg Gly Ala Arg Asp Val Ile Gln Val Val Ile Asp His Asn
           210                 215                 220
   gtg ggg gcg ggc gtc att acc gat ggt cat ctg cta cac gca ggc agc     1201
   Val Gly Ala Gly Val Ile Thr Asp Gly His Leu Leu His Ala Gly Ser
       225                 230                 235
   agt agt ctc gtg gaa ata ggc cac aca cag gtc gac ccg tat ggg aaa     1249
   Ser Ser Leu Val Glu Ile Gly His Thr Gln Val Asp Pro Tyr Gly Lys
   240                 245                 250                 255
   cgc tgt tat tgc ggg aat cac ggc tgc ctc gaa acc atc gcc agc gtg     1297
   Arg Cys Tyr Cys Gly Asn His Gly Cys Leu Glu Thr Ile Ala Ser Val
                   260                 265                 270
   gac agt att ctt gag ctg gca cag ctg cgt ctt aat caa tcc atg agc     1345
   Asp Ser Ile Leu Glu Leu Ala Gln Leu Arg Leu Asn Gln Ser Met Ser
               275                 280                 285
   tcg atg tta cat gga caa ccg tta acc gtg gac tca ttg tgt cag gcg     1393
   Ser Met Leu His Gly Gln Pro Leu Thr Val Asp Ser Leu Cys Gln Ala
           290                 295                 300
   gca ttg cgc ggc gat cta ctg gca aaa gac atc att acc ggg gtg ggc     1441
   Ala Leu Arg Gly Asp Leu Leu Ala Lys Asp Ile Ile Thr Gly Val Gly
       305                 310                 315
   gcg cat gtc ggg cgc att ctt gcc atc atg gtg aat tta ttt aac cca     1489
   Ala His Val Gly Arg Ile Leu Ala Ile Met Val Asn Leu Phe Asn Pro
   320                 325                 330                 335
   caa aaa ata ctg att ggc tca ccg tta agt aaa gcg gca gat atc ctc     1537
   Gln Lys Ile Leu Ile Gly Ser Pro Leu Ser Lys Ala Ala Asp Ile Leu
                   340                 345                 350
   ttc ccg gtc atc tca gac agc atc cgt cag cag gcc ctt cct gcg tat     1585
   Phe Pro Val Ile Ser Asp Ser Ile Arg Gln Gln Ala Leu Pro Ala Tyr
               355                 360                 365
   agt cag cac atc agc gtt gag agt act cag ttt tct aac cag ggc acg     1633
   Ser Gln His Ile Ser Val Glu Ser Thr Gln Phe Ser Asn Gln Gly Thr
           370                 375                 380
   atg gca ggc gct gca ctg gta aaa gac gcg atg tat aac ggt tct ttg     1681
   Met Ala Gly Ala Ala Leu Val Lys Asp Ala Met Tyr Asn Gly Ser Leu
       385                 390                 395
   ttg att cgt ctg ttg cag ggt taa cattttttaa ctgttctacc aaaatttgcg     1735
   Leu Ile Arg Leu Leu Gln Gly
   400                 405
   ctatctcaat ttgggccagg aaagcataac ttagactttc aaggttaatt attttcctgg   1795
   tttatatttg tgaagcataa cggtggagtt agtgatgctg aagcgtttct ttattaccgg   1855
   tacagacact tctgtaggga aaacggtggt ttcccgcgca ttgctacaag cgttagcctc   1915
   ccagggaaaa acggttgcgg gatataaacc cgtagcgaag gggagcaaag agacacccga   1975
   agggctgcgt aataaagatg ccctggtgtt gcagagtgtt tcaaccatcg aactgcctta   2035
   tgaagcagtt aatcctatcg cgttaagcga agaagaaagt agcgtggcgc acagttgccc   2095
   aatcaattac accctcattt caaacggcct ggcaaacctg accgaaaaag tcgatcatgt   2155
   cgtggtagaa gggactggcg gctggcgcag tctgatgaat gatttgcgtc cactctctga   2215
   atgggtagtg caggaacaac tgccggtgtt gatggttgtc ggtattcagg aaggttgcat   2275
   taaccatgca ctgctaacag ctcaggcgat c                                  2306
<210> 2
   <211> 406
   <212> PRT
   <213> Escherichia coli
<400> 2
<210> 3
   <211> 20
   <212> DNA
   <213> Artificial sequence
<220>
   <221> Primer
   <222> (1)..(20)
   <223> dgsA '5'-1
<400> 3
   cgaatgtaac gctggctgaa 20
<210> 4
   <211> 20
   <212> DNA
   <213> Artificial sequence
<220>
   <221> Primer
   <222> (1)..(20)
   <223> dgsA '5'-2
<400> 4
   tccagcaatg gcaagtcatc 20
<210> 5
   <211> 20
   <212> DNA
   <213> Artificial sequence
<220>
   <221> Primer
   <222> (1)..(20)
   <223> dgsA '3'-1
<400> 5
   cagcacatca gcgttgagag 20
<210> 6
   <211> 20
   <212> DNA
   <213> Artificial sequence
<220>
   <221> Primer
   <222> (1)..(20)
   <223> dgsA '3'-2
<400> 6
   gatcgcctga gctgttagca 20
<210> 7
   <211> 1756
   <212> DNA
   <213> Escherichia coli
<220>
   <221> misc_feature
   <222> (1)..(1756)
   <223>
<220>
   <221> misc_feature
   <222> (1)..(60)
   <223> Technical DNA/ remainder polylinker sequence
<220>
   <221> misc_feature
   <222> (61)..(921)
   <223> Part of the upstream-lying region and part of the 5'-region of the dgsA gene
<220>
   <221> misc_feature
   <222> (922)..(986)
   <223> Technical DNA/ remainder polylinker sequence
<220>
   <221> misc_feature
   <222> (987)..(1704)
   <223> Part of the 3'-region of the dgsA gene and part of the downstream-lying region
<220>
   <221> misc_feature
   <222> (1705)..(1756)
   <223> Technical DNA/ remainder polylinker sequence
<400> 7
   agcttggtac cgagctcgga tccactagta acggccgcca gtgtgctgga attcgccctt      60
   cgaatgtaac gctggctgaa ctggcgaaag aaccctttgt cttttttgat ccgcacgtcg    120
   ggacagggct gtatgacgat attctcgggc tgatgcgacg ttaccatttg acgcccgtca    180
   tcactcagga ggtgggcgag gcaatgacca tcatcggtct ggtttccgcc ggtctgggtg    240
   tttcaatttt gcctgcgtca tttaaacgtg ttcagctcaa cgaaatgcgc tgggtgccga    300
   ttgctgaaga ggatgcggtt tctgaaatgt ggttggtctg gccgaaacat catgaacaaa    360
   gtccggctgc gcgtaacttt cgtattcatc tgctgaatgc tctcaggtga gggaaatttc    420
   agcgaaaaag cccgaaaaat gtgctgttaa tcacatgcct aagtaaaaat ttgacgacac    480
   gtattgaagt gcttcaccat agcctacaga ttatttcgga gcgcgaaaat atagggagta    540
   tgcggtggtt gctgaaaacc agcctgggca cattgatcaa ataaagcaga ccaacgcggg    600
   cgcggtttat cgcctgattg atcagcttgg tccagtctcg cgtatcgatc tttcccgtct    660
   ggcgcaactg gctcctgcca gtatcactaa aattgtccgt gagatgctcg aagcacacct    720
   ggtgcaagag ctggaaatca aagaagcggg gaaccgtggc cgtccggcgg tggggctggt    780
   ggttgaaact gaagcctggc actatctttc tctgcgcatt agtcgcgggg agattttcct    840
   tgctctgcgc gatctgagca gcaaactggt ggtggaagag tcgcaggaac tggcgttaaa    900
   agatgacttg ccattgctgg aaagggcgaa ttctgcagat ctcggatcca ctagtaacgg    960
   ccgccagtgt gctggaattc gcccttcagc acatcagcgt tgagagtact cagttttcta   1020
   accagggcac gatggcaggc gctgcactgg taaaagacgc gatgtataac ggttctttgt   1080
   tgattcgtct gttgcagggt taacattttt taactgttct accaaaattt gcgctatctc   1140
   aatttgggcc aggaaagcat aacttagact ttcaaggtta attattttcc tggtttatat   1200
   ttgtgaagca taacggtgga gttagtgatg ctgaagcgtt tctttattac cggtacagac   1260
   acttctgtag ggaaaacggt ggtttcccgc gcattgctac aagcgttagc ctcccaggga   1320
   aaaacggttg cgggatataa acccgtagcg aaggggagca aagagacacc cgaagggctg   1380
   cgtaataaag atgccctggt gttgcagagt gtttcaacca tcgaactgcc ttatgaagca   1440
   gttaatccta tcgcgttaag cgaagaagaa agtagcgtgg cgcacagttg cccaatcaat   1500
   tacaccctca tttcaaacgg cctggcaaac ctgaccgaaa aagtcgatca tgtcgtggta   1560
   gaagggactg gcggctggcg cagtctgatg aatgatttgc gtccactctc tgaatgggta   1620
   gtgcaggaac aactgccggt gttgatggtt gtcggtattc aggaaggttg cattaaccat   1680
   gcactgctaa cagctcaggc gatcaagggc gaattctgca gatatccatc acactggcgg   1740
   ccgctcgagc atgcat                                                     1756
<210> 8
   <211> 559
   <212> DNA
   <213> Escherichia coli
<220>
   <221> misc_feature
   <222> (1)..(559)
   <223>
<220>
   <221> misc_feature
   <222> (1)..(3)
   <223> Start codon of the delta dgsA allele
<220>
   <221> misc_feature
   <222> (1)..(377)
   <223> 5'-region of the delta dgsA allele
<220>
   <221> misc_feature
   <222> (378)..(442)
   <223> Technical DNA/ remainder polylinker sequence
<220>
   <221> misc_feature
   <222> (443)..(556)
   <223> 3'-region of the delta dgsA allele
<220>
   <221> misc_feature
   <222> (557)..(559)
   <223> Stop codon of the delta dgsA allele
<400> 8
   gtggttgctg aaaaccagcc tgggcacatt gatcaaataa agcagaccaa cgcgggcgcg      60
   gtttatcgcc tgattgatca gcttggtcca gtctcgcgta tcgatctttc ccgtctggcg     120
   caactggctc ctgccagtat cactaaaatt gtccgtgaga tgctcgaagc acacctggtg     180
   caagagctgg aaatcaaaga agcggggaac cgtggccgtc cggcggtggg gctggtggtt     240
   gaaactgaag cctggcacta tctttctctg cgcattagtc gcggggagat tttccttgct     300
   ctgcgcgatc tgagcagcaa actggtggtg gaagagtcgc aggaactggc gttaaaagat     360
   gacttgccat tgctggaaag ggcgaattct gcagatctcg gatccactag taacggccgc     420
   cagtgtgctg gaattcgccc ttcagcacat cagcgttgag agtactcagt tttctaacca     480
   gggcacgatg gcaggcgctg cactggtaaa agacgcgatg tataacggtt ctttgttgat     540
   tcgtctgttg cagggttaa                                                  559